# EUROPÄISCHE PATENTSCHRIFT

(11) **EP 2 417 100 B1**
(45) Veröffentlichungstag und Bekanntmachung des Hinweises auf die Patenterteilung: **12.07.2017**
(21) Anmeldenummer: 10715181.3
(22) Anmeldetag: 07.04.2010
(51) Int. Cl.: C07C 263/10, C07C 265/00

(54) **VERFAHREN ZUR HERSTELLUNG VON ISOCYANATEN**
METHOD FOR PRODUCING ISOCYANATES
PROCÉDÉ DE PRÉPARATION D'ISOCYANATES

(30) Priorität: 08.04.2009 EP 09157627
(43) Veröffentlichungstag der Anmeldung: 15.02.2012
(73) Patentinhaber: BASF SE, 67056 Ludwigshafen am Rhein (DE)
(72) Erfinder: LEHR, Vanessa Simone, 68199 Mannheim (DE); KNOESCHE, Carsten, 67150 Niederkirchen (DE); MATTKE, Torsten, 67251 Freinsheim (DE)
(86) Internationale Anmeldenummer: PCT/EP2010/054561
(87) Internationale Veröffentlichungsnummer: WO 2010/115908

(56) Entgegenhaltungen:
- EP-A1- 1 403 248
- WO-A1-2008/055899
- WO-A1-2008/055904

## Beschreibung

Die Erfindung betrifft ein Verfahren zur Herstellung von Isocyanaten durch Umsetzung der korrespondierenden Amine mit Phosgen, gegebenenfalls in Gegenwart eines Inertmediums, bei dem Phosgen und Amin zunächst gemischt und in einem Reaktor zu Isocyanat umgesetzt werden. Ein den Reaktor verlassendes Isocyanat und Chlorwasserstoff enthaltendes Reaktionsgas wird in einem Quench durch Zugabe eines flüssigen Quenchmediums abgekühlt, wobei ein Gemisch aus Reaktionsgas und Quenchmedium als Produktstrom entsteht.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von Chlorwasserstoff (HCl) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655 oder EP-A 1 555 258 beschrieben.

Um Folgereaktionen zu vermeiden, ist es notwendig, das Reaktionsgemisch nach Reaktionsende schnell abzukühlen. Hierzu wird zum Beispiel ein Flüssigkeitsquench eingesetzt. Ein solcher Flüssigkeitsquench ist beispielsweise in EP-A 1 403 248 oder in DE-A 10 2006 058 634 beschrieben. Das Quenchmedium, das zur Kühlung zugegeben wird, weist dabei eine Temperatur auf, die im Bereich von 50 bis 200°C liegt. Durch den eingedüsten Flüssigkeitsstrom kühlt das Reaktionsgas schnell auf Temperaturen im Allgemeinen zwischen 100 und 200°C ab. Dabei entsteht ein Zwei-Phasen-Gemisch mit einer isocyanatreichen Flüssigphase und einer isocyanatarmen Gasphase. Beide werden anschließend einer gemeinsamen oder gegebenenfalls separaten Trennstufe, zum Beispiel eine Destillationsstufe zur Trennung von Chlorwasserstoff und Phosgen auf der einen Seite und Isocyanat auf der anderen Seite zugeführt.

Nachteil des Flüssigkeitsquenches, wie er beispielsweise in der EP-A 1 403 248 oder der DE-A 10 2006 058 634 beschrieben ist, ist, dass innerhalb des Flüssigkeitsquenches Wandregionen mit geringer Flüssigkeitsbenetzung auftreten können oder der Flüssigkeitsfilm an den Wandregionen aufreißen kann, was dazu führt, dass das tröpfchenförmige Kondensat nicht schnell genug abfließt. Aufgrund der längeren Verweilzeit resultieren hieraus unerwünschte Folgereaktionen und Feststoffablagerungen an den Wandungen des Flüssigkeitsquenches. Dies führt zu Ausbeuteverlusten und wirkt sich negativ auf die Laufzeit der Anlage aus.

Verfahren zur Herstellung von Isocyanaten durch Umsetzung der korrespondierenden Amine mit Phosgen, bei denen das resultierende Reaktionsgemisch in einer Quenchzone gekühlt wird, sind auch aus WO-A 2008/055899 oder WO-A 2008/055904 bekannt. Hierbei wird das Quenchmedium jeweils so eingesprüht, dass der gesamte Querschnitt des Quenches mit einem Nebel aus Quenchmedium ausgefüllt ist.

Aufgabe der vorliegenden Erfindung ist es daher, ein Verfahren zur Herstellung von Isocyanaten durch Umsetzung der korrespondierenden Amine mit Phosgen bereitzustellen, bei dem Folgereaktionen und Feststoffablagerungen vermieden oder stark reduziert werden.

Gelöst wird die Aufgabe durch ein Verfahren zur Herstellung von Isocyanaten durch Umsetzung der korrespondierenden Amine mit Phosgen, gegebenenfalls in Gegenwart eines Inertmediums, bei dem Phosgen und Amin zunächst gemischt und in einem Reaktor zu Isocyanat umgesetzt werden, wobei ein den Reaktor verlassendes Isocyanat und Chlorwasserstoff enthaltendes Reaktionsgas in einem Quench durch Zugabe eines flüssigen Quenchmediums abgekühlt wird, wobei ein Gemisch aus Reaktionsgas und Quenchmedium als Produktstrom entsteht, wobei die Wandungen des Quenchs mit einer Flüssigkeit im Wesentlichen vollständig benetzt werden.

Durch die vollständige Benetzung der Wandungen des Quenchs mit einer Flüssigkeit wird vermieden, dass sich ein tröpfchenförmiges Kondensat an den Wandungen bildet, das zu langsam abfließt und so zu Folgereaktionen und Feststoffablagerungen führt. Durch die Benetzung der Wandungen mit einer Flüssigkeit wird das an den Wandungen auskondensierende Reaktionsgemisch mit der Flüssigkeit aus dem Quench ausgetragen. Es bilden sich keine Feststoffablagerungen an den Wandungen des Quenches aus.

"Im Wesentlichen vollständig benetzt" im Rahmen der vorliegenden Erfindung bedeutet, dass alle Wandungen des Flüssigkeitsquenches, die mit dem Reaktionsgemisch in Kontakt kommen können, mit der Flüssigkeit benetzt werden. Lediglich die Bereiche des Quenches, in denen keine Benetzung mit dem Reaktionsgemisch erfolgen kann, brauchen nicht benetzt zu werden.

Zur Herstellung des Isocyanates werden das Phosgen und das Amin vorzugsweise zunächst einer Mischzone zugeführt, in der die Vermischung von Amin und Phosgen zu einem Reaktionsgemisch erfolgt. Anschließend wird das Reaktionsgemisch dem Reaktor zugeführt, in dem die Umsetzung zum Isocyanat erfolgt. Die Umsetzung von Amin und Phosgen im Reaktor erfolgt dabei vorzugsweise in der Gasphase. Hierzu liegt der Druck im Reaktor vorzugsweise im Bereich zwischen 0,3 bis 3 bar absolut, besonders bevorzugt im Bereich von 0,8 bis 3,0 bar absolut. Die Temperatur liegt dabei vorzugsweise im Bereich von 250 bis 550°C, insbesondere im Bereich von 300 bis 500°C.

Um die Reaktion in der Gasphase durchführen zu können, ist es weiterhin bevorzugt, das Amin und das Phosgen gasförmig zuzugeben. Hierzu weist das Amin vorzugsweise eine Temperatur im Bereich von 200 bis 400°C auf. Der Druck des zugegebenen Amins liegt dabei vorzugsweise im Bereich zwischen 0,05 bis 3 bar absolut. Die Temperatur des zugegebenen Phosgens liegt vorzugsweise im Bereich von 250 bis 450°C. Hierzu wird das Phosgen üblicherweise vor Zugabe auf dem Fachmann bekannte Weise erwärmt.

Zur Erwärmung des Phosgens und des Amins und zur Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur durch Absenkung des Drucks des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311°C. Es ist jedoch auch möglich, Wasserdampf mit einer Temperatur von mehr als 311 °C zur Verdampfung des Amins einzusetzen.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Der Reaktor, der zur Phosgenierung des Amins zur Herstellung von Isocyanaten eingesetzt wird, ist dem Fachmann bekannt. Im Allgemeinen werden als Reaktor Rohrreaktoren eingesetzt. Im Reaktor wird das Amin mit dem Phosgen zum korrespondierenden Isocyanat und Chlorwasserstoff umgesetzt. Üblicherweise wird das Phosgen in Überschuss zugegeben, so dass das im Reaktor entstehende Reaktionsgas neben dem gebildeten Isocyanat und dem Chlorwasserstoff auch Phosgen enthält.

Neben dem Einsatz eines Rohrreaktors ist es auch möglich, im Wesentlichen quaderförmige Reaktionsräume, beispielsweise Plattenreaktoren zu verwenden. Auch jeder beliebige andere Querschnitt des Reaktors ist möglich.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Amine und Isocyanate können aliphatisch, cycloaliphatisch oder aromatisch sein. Bevorzugt sind die Amine aliphatisch oder cycloaliphatisch, besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylen-di(phenylisocyanat) (MDI), 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, 2-Methyl-1,5-diisocyanatopentan, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0²⁶]-decan-Isomerenge-mische, sowie cycloaliphatische Diisocyanate wie 1,4-, 1,3- oder 1,2-Diisocyanato-cyclohexan, 4,4'- oder 2,4'-Di(isocyanatocyclohexyl)methan, 1-Isocyanato-3,3,5-tri-methyl-5-(isocyanatomethyl)cyclohexan (Isophorondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyana-to-3,3,5-trimethyl-5-(isocyanatomethyl)cyclohexan und 4,4'-Di(isocyanatocyclohexyl)-methan. Besonders bevorzugt sind 1,6-Diisocyanatohexan, 1,5-Diisocyanatopentan, 1-Isocyanato-3,3,5-trimethyl-5-(isocyanatomethyl)cyclohexan und 4,4'-Di(isocyanatocyclohexyl)methan.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-aminomethyl-cyclohexan (IPDA) und 4,4-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4'- und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Auftreten der Aminhydrochloride, die sich aus freien Aminogruppen und Chlorwasserstoff bilden, da die resultierenden Aminhydrochloride leicht ausfallen und nur schwer wieder verdampfbar sind.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphthalin, Decahydronaphtalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas unmittelbar nach der Reaktion in einem Quench abgekühlt. Hierzu wird ein vorzugsweise flüssiges Quenchmedium zugegeben. Durch Verdampfung des Quenchmediums nimmt dieses Wärme auf und führt zu einer schnellen Abkühlung des Reaktionsgases.

Erfindungsgemäß enthält das Quenchmedium einen Teil des im Quench abgekühlten Produktstromes. Dieser enthält zur Abkühlung eingesetztes Lösungsmittel und bei der Reaktion gebildetes Isocyanat.

Um zu vermeiden, dass sich in Rohrleitungen, Regeleinrichtungen und sonstigen Apparateteilen, insbesondere in den Zerstäuberdüsen des Quenchs Ablagerungen bilden, werden gegebenenfalls im Quenchmedium enthaltene Feststoffpartikel vor Zugabe in den Quench entfernt.

Um die Wandungen des Quenches mit einer Flüssigkeit zu benetzen, wird in einer ersten Ausführungsform die Flüssigkeit, mit der die Wandungen benetzt werden, mit Düsen an die Wandung gesprüht. Alternativ ist es auch möglich, die Flüssigkeit, mit der die Wandungen benetzt werden, beispielsweise über einen Überlauf oder beliebige andere Zuläufe, zum Beispiel Löcher oder Schlitze, zuzuführen.

Wenn die Flüssigkeit, mit der die Wandungen benetzt werden, mit Düsen an die Wandungen gesprüht wird, so kann die Flüssigkeit von oben und/oder von unten in den Quench eingesprüht werden.

Wenn die Flüssigkeit beispielsweise von oben in den Quench eingesprüht wird, weist die eingesprühte Flüssigkeit eine Geschwindigkeitskomponente in Strömungsrichtung des Reaktionsgases und eine Geschwindigkeitskomponente quer zur Strömungsrichtung des Reaktionsgases auf. Bei einem Einsprühen der Flüssigkeit von unten weist die in den Quench eingesprühte Flüssigkeit, mit der die Wandungen benetzt werden, eine Geschwindigkeitskomponente quer zur Strömungsrichtung des Reaktionsgases und eine Geschwindigkeitskomponente entgegen der Strömungsrichtung des Reaktionsgases auf.

Um die Wandungen des Quenches mit einer Flüssigkeit vollständig benetzten zu können, ist die Menge der eingespritzten Flüssigkeit so bemessen, dass nur ein Teil der Flüssigkeit im Quench verdampft und ein ausreichend großer Teil nicht verdampft, um eine vollständige Benetzung der Wandungen zu erzielen.

Wenn die Flüssigkeit über Düsen an die Wandung gesprüht wird, ist es möglich, über die gleichen Düsen sowohl die Flüssigkeit, mit der die Wandungen benetzt werden, als auch das Quenchmedium einzusprühen. Dabei können die Flüssigkeit, mit der die Wandungen benetzt werden und das Quenchmedium als Mischung über die gleichen Düsen in den Quench eingesprüht werden. Es können jedoch auch beispielsweise Zweistoffdüsen eingesetzt werden, durch die einmal die Flüssigkeit, mit der die Wandungen benetzt werden, und andererseits das Quenchmedium eingesprüht werden. Wenn das Quenchmedium und die Flüssigkeit, mit der die Wandungen benetzt werden, über die gleichen Düsen oder über Zweistoffdüsen eingesprüht werden, erfolgt das Einsprühen vorzugsweise von oben. Bevorzugt ist jedoch das Einsprühen des Quenchmediums und der Flüssigkeit, mit der die Wandungen benetzt werden, über unterschiedliche Düsen.

Weiterhin ist es auch möglich, die Flüssigkeit, mit der die Wandungen benetzt werden, sowohl von oben als auch von unten in den Quench einzusprühen. In diesem Fall ist es möglich, zum Beispiel über die Düsen, die die Flüssigkeit von oben einsprühen, gleichzeitig auch das Quenchmedium mit einzusprühen und über die Düsen, die die Flüssigkeit von unten einsprühen, die Flüssigkeit, mit der die Wandungen benetzt werden. Es ist jedoch auch hier möglich, auch über die Düsen, die die Flüssigkeit von unten einsprühen, das Quenchmedium mit einzusprühen.

Das Quenchmedium und die Flüssigkeit, mit der die Wandungen benetzt werden, können auch aus unterschiedlichen Richtungen eingesprüht werden. Das Einsprühen des Quenchmediums und der Flüssigkeit, mit der die Wandungen benetzt werden, kann dabei mit der Strömungsrichtung des Reaktionsgases, entgegen der Strömungsrichtung des Reaktionsgases oder in einem beliebigen Winkel zur Strömungsrichtung des Reaktionsgases erfolgen.

Eine gleichmäßige Flüssigkeitsbenetzung und ausreichende Filmdicke der Flüssigkeit an den Wandungen kann durch eine geeignete Oberflächenstrukturierung zur Stauung der Flüssigkeit an den Wandungen unterstützt werden.

In einer weiteren, alternativen Ausführungsform sind die zu benetzenden Wandungen porös gestaltet und die Flüssigkeit, mit der die Wandungen benetzt werden, wird durch die Poren der Wandung gedrückt. Das Drücken der Flüssigkeit durch die Poren erfolgt zum Beispiel aus einem äußeren Vorratsgefäß mit höherem Druck. Auch ist es möglich, die Wandung als Doppelmantel zu gestalten und die Flüssigkeit über den Doppelmantel durch die Poren der Wandung zu drücken. Durch das Eindrücken der Flüssigkeit durch die poröse Wandung wird erreicht, dass die Wandung im Wesentlichen vollständig mit der Flüssigkeit benetzt ist. Das Eindrücken der Flüssigkeit durch die poröse Wandung kann dabei alternativ oder zusätzlich zur Zugabe der Flüssigkeit über Düsen, einen Überlauf oder andere Zuläufe erfolgen.

Die Flüssigkeit, mit der die Wandungen benetzt werden, enthält in einer Ausführungsform mindestens einen Leichtsieder und/oder mindestens eine höher siedende Flüssigkeit, wobei unter höher siedender Flüssigkeit jede Flüssigkeit verstanden wird, die zur Benetzung der Wandung eingesetzt werden kann und nicht vollständig verdampft.

Wenn die Flüssigkeit einen Leichtsieder enthält, ist es bevorzugt, wenn der Leichtsieder und das Quenchmedium die gleiche Flüssigkeit sind. Insbesondere ist es bevorzugt, wenn der Leichsieder und das Quenchmedium ein Isocyanat und/oder mindestens ein Lösungsmittel enthalten.

Wenn als Leichtsieder und Quenchmedium unterschiedliche Flüssigkeiten eingesetzt werden, so ist es zum Beispiel möglich, als Leichtsieder ein Lösungsmittel einzusetzen und als Quenchmedium eine Flüssigkeit, die ein Isocyanat und gegebenenfalls mindestens ein Lösungsmittel enthält.

Wenn der Leichtsieder und/oder das Quenchmedium ein Isocyanat enthalten, ist es weiterhin bevorzugt, wenn das Isocyanat, das im Leichtsieder beziehungsweise im Quenchmedium enthalten ist, das gleiche ist, wie das in der Reaktion hergestellte Isocyanat. Wenn ein Isocyanat im Leichtsieder und/oder im Quenchmedium enthalten ist, so ist es insbesondere bevorzugt, wenn das bei der Reaktion gebildete Isocyanat zunächst im Quench und gegebenenfalls im nachfolgenden Kühlstufen abgekühlt wird und nach der Abkühlung ein Teilstrom als Flüssigkeit, die die Wandungen benetzt, beziehungsweise als Quenchmedium eingesetzt wird.

Auch ist es möglich, als Flüssigkeit, die die Wandungen benetzt, beziehungsweise als Leichtsieder einen Teil des den Quench verlassenen Stoffgemischs einzusetzen. Dieser kann neben dem Isocyanat zusätzlich gegebenenfalls eingesetztes Lösungsmittel sowie gegebenenfalls Reste an Phosgen und Chlorwasserstoff enthalten.

Wenn ein Quenchmedium eingesetzt wird, das von der Flüssigkeit, die die Wandungen benetzt, verschieden ist oder das Quenchmedium und die Flüssigkeit, mit der die Wandungen benetzt werden, über unterschiedliche Zugabestellen zugeführt werden, ist es bevorzugt, wenn das Quenchmedium flüssig zugegeben wird, um eine schnelle Abkühlung des Reaktionsgases im Quench zu erzielen. Die Temperatur des Quenchmediums liegt dabei vorzugsweise im Bereich von 0 bis 250°C, insbesondere im Bereich von 20 bis 220°C. Durch das Einbringen des Quenchmediums in das heiße Reaktionsgas wird das Quenchmedium erwärmt und/oder verdampft. Die für das Erwärmen und die Verdampfung des Quenchmediums notwendige Wärme wird dem Reaktionsgas entzogen und das Reaktionsgas wird auf diese Weise abgekühlt. Die Temperatur, auf die das Reaktionsgas abgekühlt wird, lässt sich zum Beispiel durch die Menge und die Temperatur des zugegebenen Quenchmediums einstellen.

Um die Temperatur, mit der das Quenchmedium in den Quench zugegeben wird, falls erforderlich einzustellen, wird das Quenchmedium vorzugsweise durch einen Wärmetauscher geleitet. Je nach Eintrittstemperatur des Quenchmediums in dem Wärmetauscher kann das Quenchmedium in diesem erwärmt oder abgekühlt werden. Ein Abkühlen ist zum Beispiel dann erforderlich, wenn ein Teil des Produktstromes, der als Quenchmedium eingesetzt wird, direkt im Anschluss an den Quench entnommen wird. Ein Erwärmen kann sich zum Beispiel dann ergeben, wenn der Teil des Produktstromes, der als Quenchmedium eingesetzt wird, am Ende der Aufbereitungsstrecke entnommen wird und eine Temperatur aufweist, die niedriger ist als die gewünschte Temperatur, mit der das Quenchmedium in den Quench zugegeben werden soll. Im Allgemeinen wird es jedoch erforderlich sein, das Quenchmedium vor Zugabe in den Quench abzukühlen.

Die Temperatur, mit der die Flüssigkeit, mit der die Wandungen benetzt werden, zugegeben wird, liegt ebenfalls vorzugsweise im Bereich von 0 bis 250°C, insbesondere im Bereich von 20 bis 220°C. Wenn die Flüssigkeit, mit der die Wandungen benetzt werden, einen Teil des Reaktionsgemischs enthält, wird dieses ebenso behandelt, wie der als Quenchmedium eingesetzte Teil des Reaktionsgemischs. So ist zum Beispiel auch hier zunächst eine Erwärmung oder Abkühlung auf die vorgesehene Einsatztemperatur möglich.

Wenn das Quenchmedium und/oder die Flüssigkeit, mit der die Wandungen benetzt werden, Lösungsmittel enthält, ist es bevorzugt, dem Quenchmedium beziehungsweise der Flüssigkeit, mit der die Wandungen benetzt werden, vor Zugabe in den Quench Lösungsmittel zuzugeben. Hierdurch können Lösungsmittelverluste in der Flüssigkeit, mit der die Wandungen benetzt werden, beziehungsweise im Quenchmedium, ausgeglichen werden. Geeignete Lösungsmittel, die im Quenchmedium beziehungsweise in der Flüssigkeit, mit der die Wandungen benetzt werden, enthalten sind, sind zum Beispiel gegebenenfalls mit Halogen substituierte Kohlenwasserstoffe. Vorzugsweise ist das Lösungsmittel, das im Quenchmedium beziehungsweise in der Flüssigkeit, mit der die Wandungen benetzt werden, enthalten ist, ausgewählt aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Dimethylisophthalat, Tetrahydroforan, Dimethylformamid, Xylol, Chlornaphthalin, Decahydronaphtalin und Toluol.

Wenn Isocyanat in der Flüssigkeit, mit der die Wandungen benetzt werden beziehungsweise im Quenchmedium enthalten ist, so liegt der Anteil an Isocyanat im Bereich von 0 bis 100 Prozent. So liegt der Anteil an Isocyanat bei einem reinen Leichtsiederquench im Allgemeinen bei 0 Prozent. Entsprechend liegt der Anteil an Isocyanat in der Flüssigkeit, mit der die Wandungen benetzt werden, ebenfalls bei 0 Prozent, wenn nur Leichtsieder eingesetzt werden. Bei einem reinen Hochsiederquench liegt der Anteil an Isocyanat dagegen zum Beispiel bei 100 Prozent. Alternativ kann in einem Hochsiederquench jedoch auch ein entsprechend hoch siedendes Lösungsmittel oder Lösungsmittelgemisch, das auch Isocyanat enthalten kann, eingesetzt werden. Der Anteil an Isocyanat in der Flüssigkeit, mit der die Wandungen benetzt werden, kann ebenfalls bei 100 Prozent liegen, wenn diese nur Hochsieder enthält. Aber auch in der Flüssigkeit, mit der die Wandungen benetzt werden, können alternativ oder zusätzlich zum Isocyanat auch hoch siedende Lösungsmittel oder Lösungsmittelgemische enthalten sein.

Jedoch kann auch in einem Leichtsiederquench Isocyanat im Quenchmedium enthalten sein. Der Anteil an Isocyanat ist in einem Hochsiederquench im Allgemeinen höher als in einem Leichtsiederquench.

Es ist aber sowohl für das Quenchmedium als auch für die Flüssigkeit, mit der die Wandungen benetzt werden, jede beliebige Zusammensetzung an Lösungsmittel und Isocyanat möglich. Die Zusammensetzung hängt dabei zum Beispiel auch davon ab, an welcher Stelle im Prozess der Teil des Reaktionsgemischs, der als Quenchmedium, beziehungsweise als Flüssigkeit, die die Wandungen benetzt, eingesetzt wird, abgezweigt wird.

Wenn als Quenchmedium und als Flüssigkeit, mit der die Wandungen benetzt werden, unterschiedliche Flüssigkeiten eingesetzt werden, so ist es zum Beispiel möglich, als Quenchmedium Leichtsieder, beispielsweise Lösungsmittel, und als Flüssigkeit, mit der die Wandungen benetzt werden, eine höher siedende Substanz als den als Quenchmedium eingesetzten Leichtsieder, beispielsweise reines Isocyanat, einzusetzen. Bevorzugt werden zum Benetzen der Wandungen höher siedende Lösungsmittel oder Lösungsmittelgemische, die auch Isocyanat enthalten können, eingesetzt.

Als Hochsieder können zum Beispiel auch die oben genannten Lösungsmittel, vorzugsweise in Mischung mit Isocyanat, eingesetzt werden.

In einer bevorzugten Ausführungsform schließen sich zur weiteren Behandlung an den Quench weitere Stufen zur Abkühlung des Reaktionsgases an. In den einzelnen Stufen zur Abkühlung erfolgt dabei jeweils eine weitere Abkühlung des Produktstromes bis zum Erreichen der gewünschten Temperatur, mit der der Produktstrom beispielsweise einer nachfolgenden Aufarbeitung zugeführt wird. Als Produktstrom wird im Quench dabei vorzugsweise der gesamte, den Quench verlassende Strom eingesetzt, der sowohl das Quenchmedium, das Reaktionsgemisch als auch die Flüssigkeit, mit der die Wandungen benetzt werden, enthält.

Die weiteren Stufen zur Abkühlung, die sich an den Quench anschließen können, können zum Beispiel weitere Quenche oder Kondensatoren oder beliebige andere Stufen zur Abkühlung, die dem Fachmann bekannt sind, sein. Bevorzugt ist mindestens eine der sich an den Quench anschließenden Stufen zur Abkühlung des Produktstromes ein Kondensator. Als Kondensator eignet sich dabei jede beliebige, dem Fachmann bekannte Kondensatorbauart. Üblicherweise wird als Kondensator ein Wärmetauscher eingesetzt, der von einem Kühlmedium durchströmt wird. Als Kühlmittel kann zum Beispiel Wasser eingesetzt werden. In diesem Fall kondensiert das Gas zumindest teilweise an den Wandungen des Kondensators aus. Die so entstehende Flüssigkeit läuft ab und wird gesammelt und dem Kondensator entnommen.

Nach dem Kondensieren des Produktstromes wird im Allgemeinen eine Aufbereitung angeschlossen. So ist es zum Beispiel möglich, dass das auskondensierte Gemisch in einem Lösungsmittel gewaschen wird. Als Lösungsmittel können zum Beispiel die gleichen Stoffe eingesetzt werden, die auch als Quenchmedium beziehungsweise als Flüssigkeit, mit der die Wandungen benetzt werden, eingesetzt werden können.

Alternativ zur Abkühlung des Produktstromes ist es auch möglich, dass der Produktstrom nach Austritt aus dem Quench einer Trennstufe zugeführt wird. Eine entsprechende Trennstufe kann sich alternativ jedoch auch zum Beispiel dem Kondensator anschließen. Bevorzugt schließt sich jedoch die Trennstufe direkt an den Quench an. Als Trennstufen eignen sich zum Beispiel Destillationskolonnen oder Wäscher.

Wenn die Trennstufe ein Wäscher ist, so wird der den Quench verlassende Produktstrom vorzugsweise - wie zuvor beschrieben - mit einem Lösungsmittel gewaschen. Hierbei wird das Isocyanat selektiv in die Waschlösung überführt. An die Wäsche schließt sich dann eine Trennung, bevorzugt mittels Rektifikation, an.

Wenn die Trennstufe eine Destillationskolonne ist, so wird der gasförmige Produktstrom der Rektifikationskolonne zugeführt. Die Rektifikationskolonne wird vorzugsweise so betrieben, dass die Temperatur am Kopf der Rektifikationskolonne niedriger ist als die Siedetemperatur des Produktstromes. Auf diese Weise kondensieren in der Destillationskolonne selektiv einzelne Bestandteile des Produktstromes aus und können der Kolonne am Sumpf, über Kopf und gegebenenfalls über Seitenabzüge entnommen werden.

Wenn die Trennstufe ein Wäscher ist, so eignet sich insbesondere ein Waschturm, in dem aus dem gasförmigen Produktstrom das gebildete Isocyanat durch Kondensation in einem inerten Lösungsmittel abgetrennt wird, während überschüssiges Phosgen, Chlorwasserstoff und gegebenenfalls das Inertmedium den Waschturm gasförmig durchlaufen. Bevorzugt wird dabei die Temperatur des inerten Lösungsmittels oberhalb der Lösungstemperatur des zum Amin gehörigen Carbamoylchlorids im gewählten Waschmedium gehalten. Besonders bevorzugt wird dabei die Temperatur des inerten Lösungsmittels oberhalb der Schmelztemperatur des zum Amin gehörigen Carbamoylchlorids gehalten.

Als Wäscher eignet sich jeder beliebige, dem Fachmann bekannte Wäscher. So können zum Beispiel Rührbehälter oder andere herkömmliche Apparaturen, beispielsweise Kolonnen oder Mixer-Settler-Apparaturen eingesetzt werden.

Das Waschen und die Aufarbeitung des den Quench verlassenen Gemischs aus Reaktionsgas, Quenchmedium und Flüssigkeit, mit der die Wandungen benetzt werden, erfolgt dabei im Allgemeinen wie beispielsweise in WO-A 2007/028715 beschrieben.

Wenn zur Aufbereitung des Produktstromes ein Kondensator eingesetzt wird, ist es bevorzugt, das Quenchmedium beziehungsweise die Flüssigkeit, mit der die Wandungen benetzt werden, dem Kondensator zu entnehmen. Bei einer Aufbereitung durch Rektifikation erfolgt vorzugsweise eine Abtrennung des als Quenchmedium beziehungsweise als Flüssigkeit, mit der die Wandungen benetzt werden, eingesetzten Lösungsmittels. Das Lösungsmittel enthält in diesem Fall noch Anteile an Isocyanaten. Das so abgetrennte Gemisch aus Lösungsmittel und Isocyanat wird dann als Quenchmedium beziehungsweise als Flüssigkeit, mit der die Wandungen benetzt werden, eingesetzt.

Wenn ein Teil des Produktstromes als Quenchmedium beziehungsweise als Flüssigkeit, mit der die Wandungen benetzt werden, eingesetzt wird, so ist es möglich, diesen Teil zum Beispiel nach dem Abkühlen aus dem Produktstrom abzuzweigen. Alternativ können das Quenchmedium beziehungsweise die Flüssigkeit, mit der die Wandungen benetzt werden, auch nach einer sich an den Quench anschließenden Aufarbeitung aus einem beliebigen Strom abgezweigt werden.

## Patentansprüche

1. Verfahren zur Herstellung von Isocyanaten durch Umsetzung der korrespondierenden Amine mit Phosgen, gegebenenfalls in Gegenwart eines Inertmediums, bei dem Phosgen und Amin zunächst gemischt und in einem Reaktor zum Isocyanat umgesetzt werden, wobei ein den Reaktor verlassendes Isocyanat und Chlorwasserstoff enthaltendes Reaktionsgas in einem Quench durch Zugabe eines flüssigen Quenchmediums abgekühlt wird, wobei ein Gemisch aus Reaktionsgas und Quenchmedium als Produktstrom entsteht, **dadurch gekennzeichnet, dass** die Wandungen des Quenchs, die mit dem Reaktionsgas in Kontakt kommen können, mit einer Flüssigkeit benetzt werden, wobei die Menge an Flüssigkeit so bemessen wird, dass nur ein Teil der Flüssigkeit im Quench verdampft und ein ausreichend großer Teil nicht verdampft, um eine vollständige Benetzung der Wandungen zu erzielen.

2. Verfahren gemäß Anspruch 1, **dadurch gekennzeichnet, dass** die Flüssigkeit, mit der die Wandungen benetzt werden, mit Düsen an die Wandung gesprüht wird.

3. Verfahren gemäß Anspruch 2, **dadurch gekennzeichnet, dass** die Flüssigkeit, mit der die Wandungen benetzt werden, von oben und/oder von unten in den Quench eingesprüht wird.

4. Verfahren gemäß Anspruch 2 oder 3, **dadurch gekennzeichnet, dass** die Flüssigkeit, mit der die Wandungen benetzt werden und das Quenchmedium als Mischung mit den gleichen Düsen in den Quench eingesprüht werden.

5. Verfahren gemäß Anspruch 2 oder 3, **dadurch gekennzeichnet, dass** das Quenchmedium und die Flüssigkeit, mit der die Wandungen benetzt werden, mit Zweistoffdüsen oder unterschiedlichen Düsen in den Quench eingesprüht werden.

6. Verfahren gemäß Anspruch 1, **dadurch gekennzeichnet, dass** die Flüssigkeit, mit der die Wandungen benetzt werden, über einen Überlauf oder andere Zuläufe bereitgestellt wird.

7. Verfahren gemäß einem der Ansprüche 1 bis 6, **dadurch gekennzeichnet, dass** die Flüssigkeit, mit der die Wandungen benetzt werden, mindestens einen Leichtsieder und/oder mindestens einen Hochsieder enthält.

8. Verfahren gemäß Anspruch 7, **dadurch gekennzeichnet, dass** die Flüssigkeit, mit der die Wandungen benetzt werden, und das Quenchmedium die gleiche Flüssigkeit sind.

9. Verfahren gemäß Anspruch 7 oder 8, **dadurch gekennzeichnet, dass** die Flüssigkeit, mit der die Wandungen benetzt werden, ein Isocyanat und/oder mindestens ein Lösungsmittel enthält.

10. Verfahren gemäß einem der Ansprüche 7 bis 9, **dadurch gekennzeichnet, dass** das Isocyanat, das in der Flüssigkeit, mit der die Wandungen benetzt werden, enthalten ist, das gleiche wie das in der Reaktion hergestellte Isocyanat ist.

11. Verfahren gemäß Anspruch 10, **dadurch gekennzeichnet, dass** das Reaktionsgas zunächst im Quench und gegebenenfalls nachfolgenden Kühlstufen abgekühlt wird und zu einem Produktstrom kondensiert wird und nach der Abkühlung ein Teilstrom als Flüssigkeit, die die Wandungen benetzt, eingesetzt wird.

12. Verfahren gemäß einem der Ansprüche 7 bis 11, **dadurch gekennzeichnet, dass** das Lösungsmittel ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol.

## Claims

1. A process for preparing isocyanates by reacting the corresponding amines with phosgene, optionally in the presence of an inert medium, in which phosgene and amine are first mixed and converted to the isocyanate in a reactor, and in which a reaction gas which comprises isocyanate and hydrogen chloride leaving the reactor is cooled in a quench by adding a liquid quench medium to form a mixture of reaction gas and quench medium as the product stream, which comprises wetting the walls of the quench which can come into contact with the reaction gas with a liquid, wherein the amount of liquid is such that only some of the liquid in the quench evaporates and there is a sufficiently large portion that does not evaporate in order to achieve complete wetting of the walls.

2. The process according to claim 1, wherein the liquid with which the walls are wetted is sprayed onto the wall with nozzles.

3. The process according to claim 2, wherein the liquid with which the walls are wetted is sprayed into the quench from the top and/or from the bottom.

4. The process according to claim 2 or 3, wherein the liquid with which the walls are wetted and the quench medium are sprayed into the quench as a mixture with the same nozzles.

5. The process according to claim 2 or 3, wherein the quench medium and the liquid with which the walls are wetted are sprayed into the quench with two-substance nozzles or different nozzles.

6. The process according to claim 1, wherein the liquid with which the walls are wetted is provided via an overflow or other feeds.

7. The process according to any of claims 1 to 6, wherein the liquid with which the walls are wetted comprises at least one low boiler and/or at least one high boiler.

8. The process according to claim 7, wherein the liquid with which the walls are wetted and the quench medium are the same liquid.

9. The process according to claim 7 or 8, wherein the liquid with which the walls are wetted comprises an isocyanate and/or at least one solvent.

10. The process according to any of claims 7 to 9, wherein the isocyanate present in the liquid with which the walls are wetted is the same as the isocyanate prepared in the reaction.

11. The process according to claim 10, wherein the reaction gas is first cooled in the quench and any downstream cooling stages and is condensed to a product stream and, after the cooling, a substream is used as the liquid which wets the walls.

12. The process according to any of claims 7 to 11, wherein the solvent is selected from the group consisting of monochlorobenzene, dichlorobenzene, trichlorobenzene, hexane, benzene, 1,3,5-trimethylbenzene, nitrobenzene, anisole, chlorotoluene, o-dichlorobenzene, diethyl isophthalate, tetrahydrofuran, dimethylformamide, xylene, chloronaphthalene, decahydronaphthalene and toluene.

## Revendications

1. Procédé de fabrication d'isocyanates par mise en réaction des amines correspondantes avec du phosgène, éventuellement en présence d'un milieu inerte, selon lequel le phosgène et l'amine sont tout d'abord mélangés et mis en réaction dans un réacteur pour former l'isocyanate, un gaz réactionnel contenant l'isocyanate et du chlorure d'hydrogène quittant le réacteur étant refroidi dans une trempe par ajout d'un milieu de trempe liquide, un mélange du gaz réactionnel et du milieu de trempe étant formé en tant que courant de produits, **caractérisé en ce que** les parois de la trempe qui peuvent rentrer en contact avec le gaz réactionnel sont mouillées avec un liquide, la quantité de liquide étant déterminée de sorte qu'uniquement une partie du liquide s'évapore dans la trempe et une partie suffisamment importante ne s'évapore pas pour obtenir un mouillage complet des parois.

2. Procédé selon la revendication 1, **caractérisé en ce que** le liquide avec lequel les parois sont mouillées est pulvérisé sur la paroi avec des buses.

3. Procédé selon la revendication 2, **caractérisé en ce que** le liquide avec lequel les parois sont mouillées est pulvérisé dans la trempe depuis le haut et/ou depuis le bas.

4. Procédé selon la revendication 2 ou 3, **caractérisé en ce que** le liquide avec lequel les parois sont mouillées et le milieu de trempe sont pulvérisés sous la forme d'un mélange avec les mêmes buses dans la trempe.

5. Procédé selon la revendication 2 ou 3, **caractérisé en ce que** le milieu de trempe et le liquide avec lequel les parois sont mouillées sont pulvérisés dans la trempe avec des buses à deux composants ou des buses différentes.

6. Procédé selon la revendication 1, **caractérisé en ce que** le liquide avec lequel les parois sont mouillées est mis à disposition par un trop-plein ou d'autres alimentations.

7. Procédé selon l'une quelconque des revendications 1 à 6, **caractérisé en ce que** le liquide avec lequel les parois sont mouillées contient au moins un composant de point d'ébullition faible et/ou au moins un composant de point d'ébullition élevé.

8. Procédé selon la revendication 7, **caractérisé en ce que** le liquide avec lequel les parois sont mouillées et le milieu de trempe sont le même liquide.

9. Procédé selon la revendication 7 ou 8, **caractérisé en ce que** le liquide avec lequel les parois sont mouillées contient un isocyanate et/ou au moins un solvant.

10. Procédé selon l'une quelconque des revendications 7 à 9, **caractérisé en ce que** l'isocyanate qui est contenu dans le liquide avec lequel les parois sont mouillées est identique à l'isocyanate fabriqué dans la réaction.

11. Procédé selon la revendication 10, **caractérisé en ce que** le gaz réactionnel est tout d'abord refroidi dans la trempe et éventuellement dans des étapes de refroidissement ultérieures, et est condensé en un courant de produits, et un courant partiel est utilisé après le refroidissement en tant que liquide qui mouille les parois.

12. Procédé selon l'une quelconque des revendications 7 à 11, **caractérisé en ce que** le solvant est choisi dans le groupe constitué par le monochlorobenzène, le dichlorobenzène, le trichlorobenzène, l'hexane, le benzène, le 1,3,5-triméthylbenzène, le nitrobenzène, l'anisole, le chlorotoluène, l'o-dichlorobenzène, l'isophtalate de diéthyle, le tétrahydrofurane, le diméthylformamide, le xylène, la chloronaphtaline, la décahydronaphtaline et le toluène.
